# EUROPEAN PATENT SPECIFICATION

(11) **EP 0 492 383 B1**
(45) Date of publication and mention of the grant of the patent: **17.04.1996**
(21) Application number: 91121676.0
(22) Date of filing: 18.12.1991
(51) Int. Cl.: A61K 31/40

(54) **Use of heptastigmine in the therapy of cerebral ictus**
Verwendung von Heptastigmin zur Behandlung der zerebralen Ictus
Utilisation de l'heptastigmine dans le traitement de l'ictus cérébral

(30) Priority: 21.12.1990 IT 2251390
(43) Date of publication of application: 01.07.1992
(73) Proprietor: MEDIOLANUM FARMACEUTICI S.P.A., I-20143 Milano (IT)
(72) Inventor: Del Bono, Rinaldo, I-20122 Milan (IT); Imbimbo, Bruno Pietro, I-20141 Milan (IT)
(74) Representative: Gervasi, Gemma, Dr.

(56) References cited:
- EP-A- 0 154 864
- STROKE, vol. 17, no. 5, September-October 1986, pages 1004-1009; O.U. SCREMIN et al.: "Physostigmine induced reversal of ischemia following acute middle cerebral artery occlusion in the rat"
- NEUROPSYCHOPHARMACOLOGY, vol. 1, no. 4, December 1988, pages 297-303; O.U. SCREMIN et al.: "Physostigmine enhances blood flow-metabolism ratio in neocortex"
- ACTA PHYSIOLOGICA ET PHARMACOLOGICA BULGARICA, vol. 15, no. 3, 1989, pages 3-9; C. CASTELLANO et al.: "Age-determined changes in the effects of a newly synthetized cholinesterase inhibitor on exploratory behavior, memory and striatal cholinesterase activity in rats"
- PHARMACOLOGY BIOCHEMISTRY & BEHAVIOR, vol.26, no. 3, March 1987, pages 625-629; M. BRUFANI et al.: "A long-lasting cholinesterase inhibitor affecting neural and behavioral processes"

## Description

### Prior art

The importance of those clinical situations known by the term cerebral ictus is well known. Their annual incidence, limited to 5-10 per 100,000 persons below 40 years of age, rises to 10-20 per 1000 in persons older than 65 years.

Epidemiologic studies indicate that 18% of cerebral ictus cases are caused by intracranial hemorrhages and 82% by cerebral infarct determined by emboly or thrombosis.

There is currently no pharmacological treatment for reducing cerebral damage caused by cerebral ictus (Nadean S.E. Stroke. Medical Clinics of North America 1989; 73: 1351-1369).

Drugs proposed for the treatment of cerebral hemorrhage include for example the antifibrinolytics, but their use has not yet been proved effective and is discouraged by many in that they increase complications (Aminoff, Current Medical Diagnosis and Treatment, 1988).

Fibrinolytics have been proposed for treating emboly and thrombosis, although many authors are contrary to their use, whereas dicumarolytics are used for prophylactic purposes. Hence the major objectives of the medical practitioner are to prevent recurrence and optimize patient rehabilitation.

It is also known that physostigmine, a drug which inhibits acetylcholinesterase and is hence of cholinergic action, is able to increase the cerebral blood flow. This effect is however of short duration (Scremin O.U., Scremin A.M.E., Somani S.M., Giacobini E., Brain regional distribution of physostigmine and its relation to cerebral blood flow following intravenous administration in rats, Journal of Neuroscience Research 1990; 26: 188-195) and is limited to a few specific cerebral regions (Scremin O.U., Torres C.D., Scremin A.M.E., Differential effects of physostigmine on blood flow-metabolism coupling in cerebral cortex and superior colliculus. Journal of Cerebral Blood Flow and Metabolism 1987; 7: S264, and Scremin O.U., Allen K., Torres C.D., Scremin A.M.E., Physostigmine enhances blood flow metabolism ratio in neocortex, Neuropsychopharmacology 1988; 1: 297-303). In addition, physostigmine has poor and erratic absorption following oral administration and has considerable systemic toxicity, so that its clinical use is very limited. It is also known that in the treatment of Alzheimer's disease, a senile degenerative form of the brain accompanied by a significant reduction in the cerebral concentration of acetylcholine, heptastigmine is potentially preferable to physostigmine in that it has lesser toxicity and greater duration of action (Italian patent application 47780 A/84).

### Summary

We have now found that heptastigmine increases the cerebral blood flow in rats in a significant and prolonged manner at well tolerated doses. This implies that the cerebral damage caused by the cerebral ictus can be reduced by administering heptastigmine, which hence constitutes a drug for novel use in the treatment of this pathology.

The present invention therefore relates to the use of heptastigmine in the preparation of pharmaceutical compositions for the pharmacological treatment of cerebral ictus.

### Detailed description of the invention

The advantages of the use of heptastigmine in the preparation of pharmaceutical compositions for the pharmacological treatment of cerebral ictus according to the present invention will be more apparent from the following detailed description relating to the effect of heptastigmine on the blood flow in the various regions of the brain.

The pharmacological trials were conducted on Wistar rats weighing 250-300 g, determination of the regional cerebral blood flow (rCBF) being by the iodo[¹⁴C] antipyrine (IAP) method described by Sakurada et al., (Sakurada O., Kennedy C., Jehle J., Brown J.D., Carbin G.L., Sokoloff L., Measurement of local cerebral blood flow with iodo[¹⁴C] antipyrine. American Journal of Physiology 1978; 234: H59-H66).

Two arterial catheters and two venous catheters were inserted into the femoral veins of rats anesthetized with halothane.

One arterial catheter was connected to a pressure transducer to continuously record the blood pressure while the other was used to sample the arterial blood.

One venous catheter was used for IAP infusion, the other being used for infusion of a euthanasia agent (T-61, American Hoechst Corp.) on termination of the IAP infusion.

Heptastigmine tartrate, dissolved in physiological solution, was injected into the vein in doses of 0.5, 1.0, 1.5 and 3.0 mg/kg within a time of 20 seconds. The procedure for determining the rCBF was initiated at 20, 40, 90, 180 and 360 minutes after injection of the drug. The IAP (100 µCi/kg) was infused at a constant rate over a period of 30 seconds with arterial blood sampling every 2-3 seconds, followed by the euthanasia agent. The exact moment of cardiac arrest was identified by continuous recording of the arterial pressure.

The brain was removed, frozen in methylbutane at -70°C and sectioned in a cryostat at -20°C into slices 20 µm thick. These sections were dried by heating and exposed to a Kodak NMC film for two weeks together with standards of known reactivity provided by ¹⁴C polymethylmethacrylate.

The radioautographs of the brain sections and standards were digitalized, the optical densities then being measured to calculate the rCBF expressed in ml g⁻¹ min⁻¹.

In the dose-effect study a marked increase in cerebral blood flow was encountered in all regions, statistical analysis showing significant differences relative to the controls (P<0.05) for the following regions: for the corpus callosum at 0.5 mg/kg; for the anteromedial thalamic nucleus, the dorsal hypothalamic area, the superior colliculus upper layer, the superior colliculus deep layer, the reticular nucleus pontis, the cerebellum, the pineal body, the nuclei pontis, the corpus callosum and the internal capsule at 1 kg; for all regions with the exception of the CA1 field of Ammon's horn, the caudate putamen and the central nucleus of the inferior colliculus at 3 mg/kg.

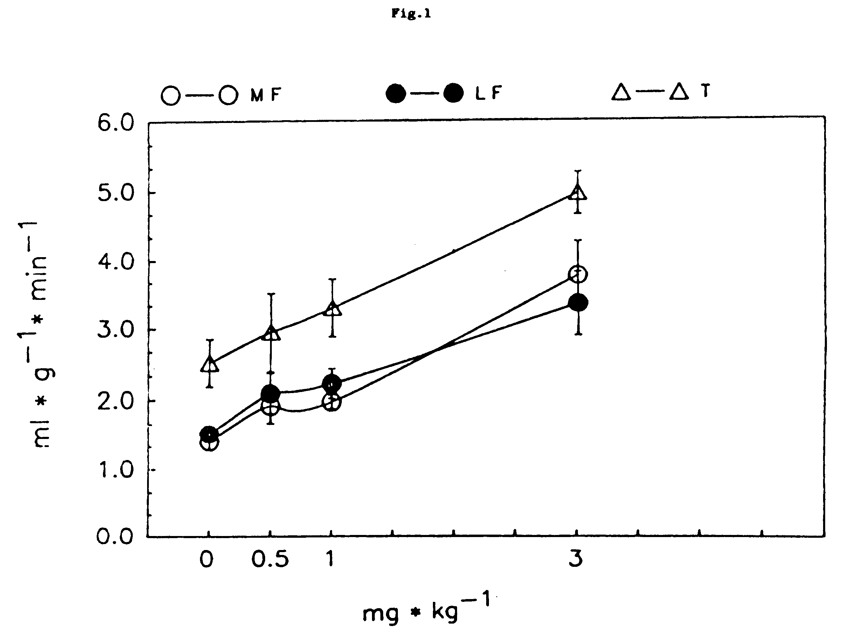

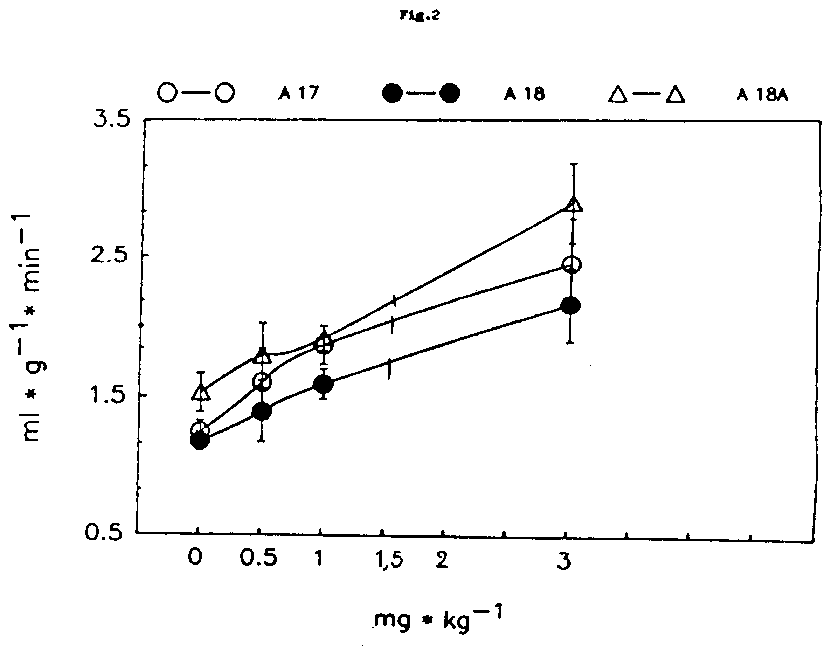

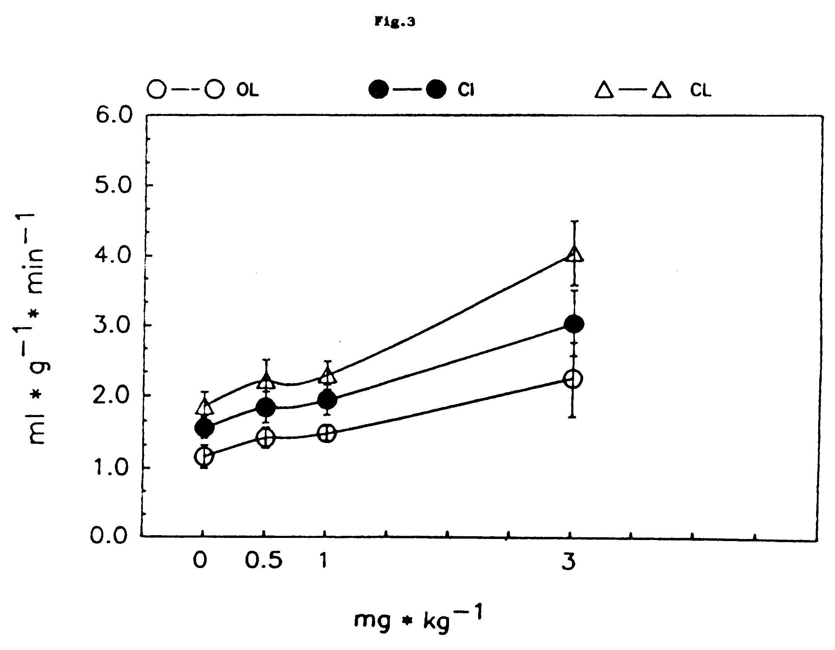

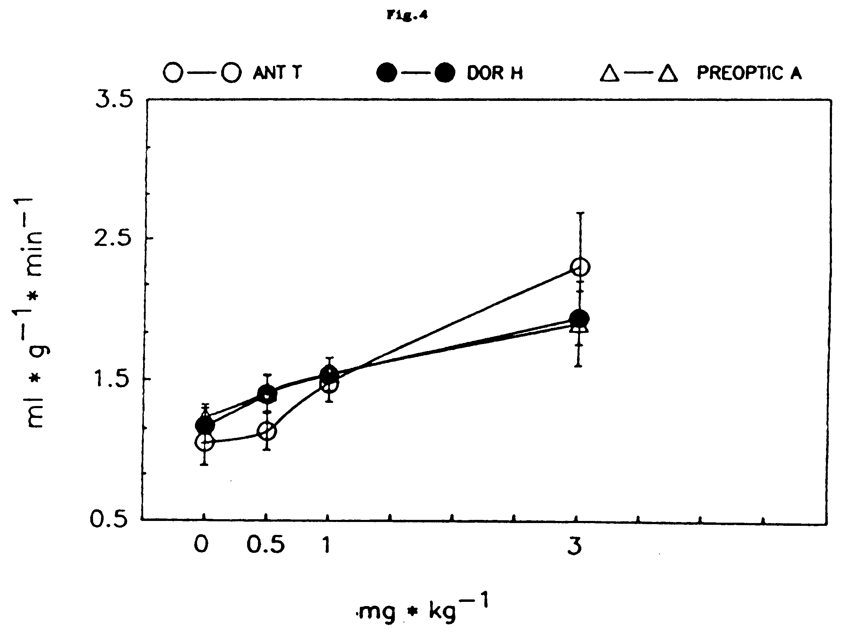

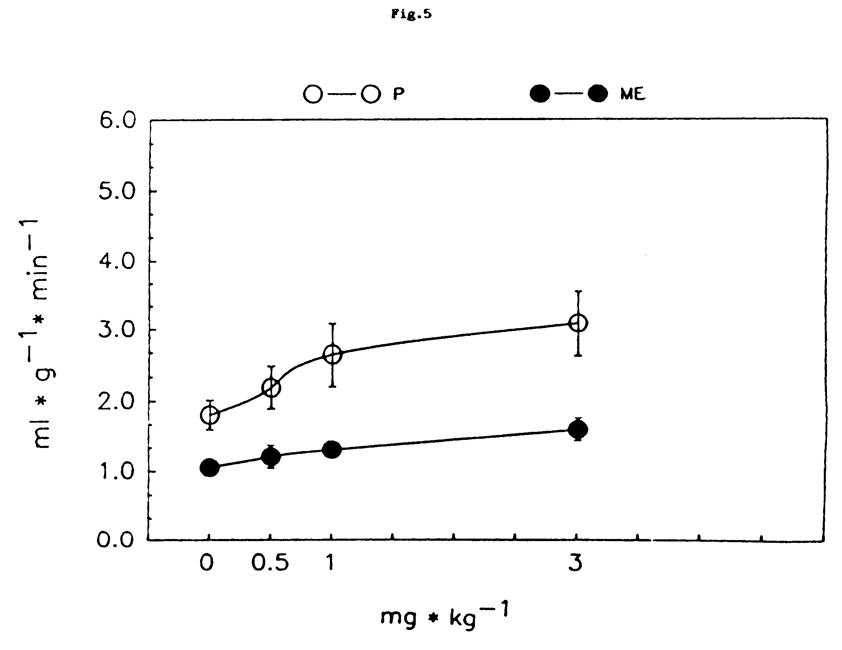

The results of the dose-effect study are shown in Figures 1 to 5 for doses of 0.5, 1.0 and 3.0 mg/kg administered intravenously. In these figures the vertical axis represents the rCBF and the horizontal axis the administered dose, the symbols at the top of the figures having the following meanings:
MF = middle frontal cortex;
LF = lateral frontal cortex;
T = temporal cortex, auditory area;
A17 = striate cortex, area 17;
A18 = striate cortex, area 18;
A18A = striate cortex, area 18A;
OL = primary olfactory cortex
CI = front cingulate cortex;
CL = claustrum;
ANT T = anteromedial thalamic nucleus;
DOR H = dorsal hypothalamic area;
PREOPTIC A = lateral preoptic area;
P = pineal body;
ME = medial eminence.

The cerebral blood flow was determined 30 min after drug administration.

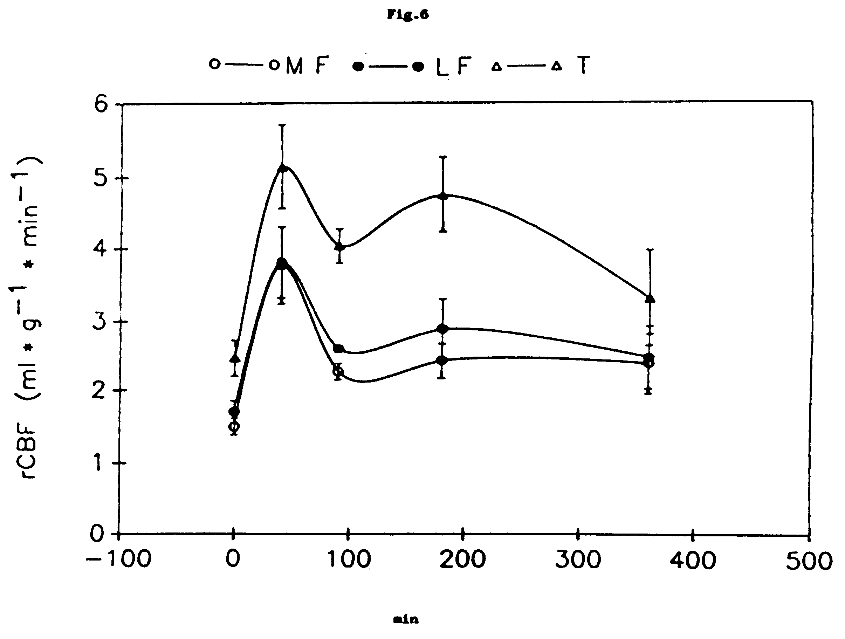

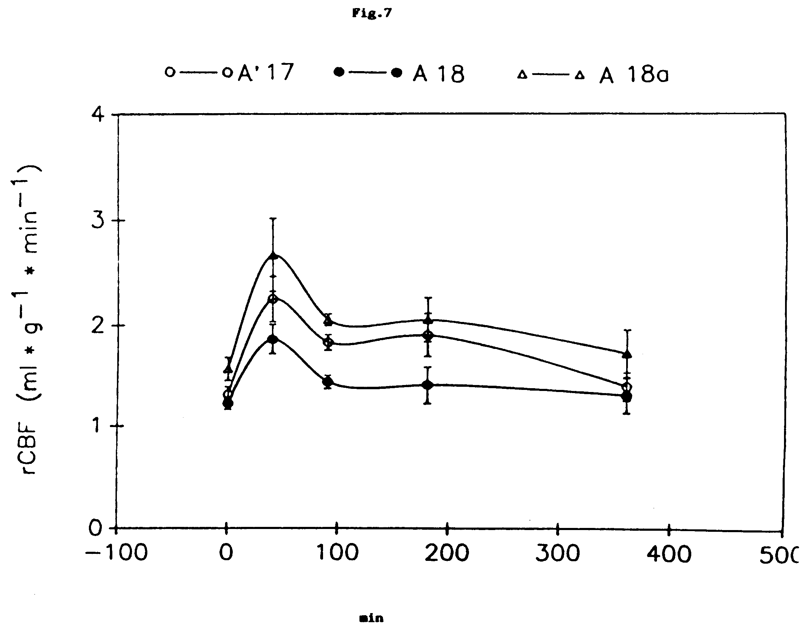

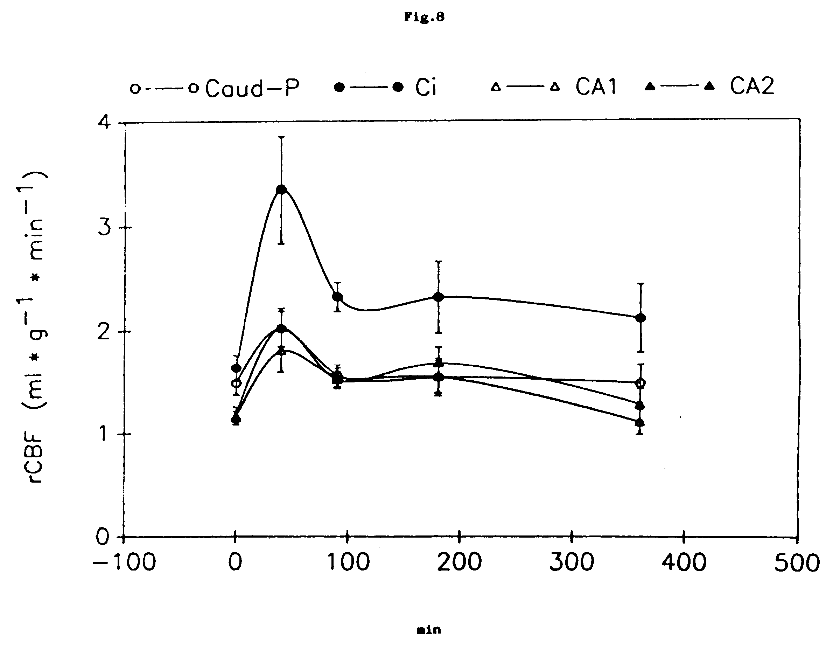

The results of the blood flow study against time are shown in Figures 6 to 8 for a single dose of heptastigmine administered intravenously (1.5 mg/kg).

The rCBF was measured at zero time and at 40, 90, 180 and 360 minutes after injecting the drug.

The symbols at the top of the figures have the following meanings:
MF = middle frontal cortex;
LF = lateral frontal cortex;
T = temporal cortex, auditory area;
A17 = striate cortex, area 17;
A18 = striate cortex, area 18;
A18a = striate cortex, area 18a;
Caud-P = caudate putamen;
Ci = front cingulate cortex;
CA1 = field CA1 of Ammon's horn;
CA2 = field CA2 of Ammon's horn.

Significant increases in blood flow (P<0.05) were observed in all regions after 40 minutes; in all regions with the exception of area 18, area 18a and the caudate putamen at 90 minutes; only in the middle frontal cortex, the temporal cortex and field CA2 of Ammon's horn after 180 minutes; and in no region after 360 minutes.

The results therefore demonstrate that heptastigmine administered intravenously induces a significant increase in blood flow in numerous cerebral regions, with a maximum at 40 minutes after injection, the effect still being statistically significant up to three hours after injection.

The regional distribution of the blood flow increase is wider and is of longer duration than that observed with physostigmine (Scremin O.U., Allen K., Torres C.D., Scremin A.M.E., Physostigmine enhances blood flow metabolism ratio in neocortex, Neuropsychopharmacology 1988; 1: 297-303 and Scremin O.U., Scremin A.M.E., Somani S.M., Giacobini E., Brain regional distribution of physostigmine and its relation to cerebral blood flow following intravenous administration in rats, Journal of Neuroscience Research 1990; 26: 188-195).

A further confirmation of the effectiveness of heptastigmine in the treatment of the cerebral ictus is given by the variation of the cerebral blood flow (CBF) as a function of the cerebral glucose use (CGU).

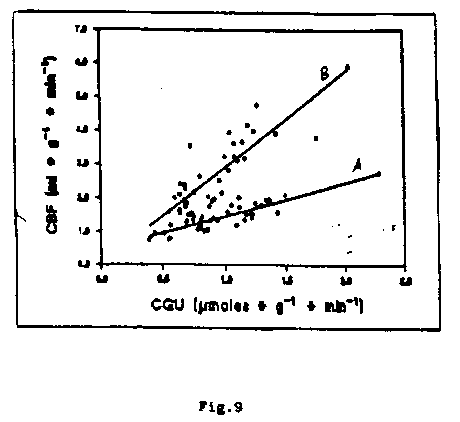

The graphic representation of said variation is illustrated in Fig. 9 wherein curve A refers to rats being treated with a physiological solution (comparisons) and curve B refers to rats being treated with heptastigmine.

The cerebral blood flow was measured by means of Iodo- ¹⁴C antipyrine and the cerebral glucose use was determined by ¹⁴C-2-deoxyglucose. Each point represents the average of blood flow and of glucose use in 5 rats in 38 cerebral regions.

These results show that the administration of heptastigmine provides an increase of cerebral blood flow without requiring further energy.

Toxic effects of the drug were not observed at the doses used.

The abovementioned results show that heptastigmine exercizes a prolonged action useful in improving cerebral ischemia and hypoxia situations, so that it can be used successfully in cerebral ictus therapy. The present invention therefore provides for its use in the preparation of pharmaceutical compositions for the therapeutic treatment of cerebral ictus, which by increasing the cerebral blood flow prevent cerebral damage caused by cerebral ictus.

Said compositions consist of heptastigmine preferably in the form of a soluble salt such as tartrate, citrate and maleate, together with pharmacologically acceptable and compatible solvents and diluents such as physiological solution and distilled water.

## Claims

1. The use of heptastigmine in the preparation of pharmaceutical compositions for the therapeutic treatment of cerebral ictus.

2. The use claimed in claim 1, characterised in that said heptastigmine is in the form of a soluble salt chosen from the group consisting of tartrate, citrate and maleate.

## Patentansprüche

1. Die Verwendung von Heptastigmin bei der Herstellung von pharmazeutischen Zusammensetzungen für die therapeutische Behandlung des zerebralen Insults.

2. Die im Anspruch 1 beanspruchte Verwendung, dadurch gekennzeichnet, daß das genannte Heptastigmin in der Form eines löslichen Salzes, ausgewählt aus der Gruppe, die aus dem Tartrat, Citrat und Maleat besteht, vorliegt.

## Revendications

1. L'utilisation de l'heptastigmine dans la préparation de compositions pharmaceutiques pour le traitement thérapeutique de l'ictus cérébral.

2. L'utilisation selon la revendication 1, caractérisée en ce que ladite heptastigmine est sous forme d'un sel soluble choisi dans le groupe constitué par le tartrate, le citrate et le maléate.
